# EUROPEAN PATENT APPLICATION

(11) **EP 4 793 915 A1**
(43) Date of publication of application: **19.08.2026**
(21) Application number: 25157747.4
(22) Date of filing: 13.02.2025
(51) Int. Cl.: G07F 11/26, G07F 11/68, G07F 17/00

(54) **DEVICE FOR CONVEYING MEDICATION SACHETS**

(71) Applicant: Evondos Oy, 24240 Salo (FI)
(72) Inventor: CRICHTON, Daniel, Cambridge, CB4 1HE (GB); MAUND, Alexander, Surrey, KT13 8LB (GB); MACLACHLAN, Alex, Suffolk, SO14 4JX (GB); GRIMWADE, Steve, Lincolnshire, PE12 0TU (GB)
(74) Representative: Berggren Oy

(57) **Abstract**

The present invention relates to a device (100) for conveying medication sachets (201). The device (100) comprises an inlet (110) for receiving the medication sachets (201) to be conveyed, an outlet (114) for discharging the conveyed medication sachets (201), a first endless belt (101), a plurality of first guide elements (103) arranged to guide the first endless belt (101) along a first path, of which first guide elements at least one is a first guide roller (103), a second endless belt (102), a plurality of second guide elements (104) arranged to guide the second endless belt (102) along a second path, of which second guide elements at least one is a second guide roller (104), a plurality of third guide rollers (105) arranged to guide the first endless belt (101) and the second endless belt (102) along a third path, wherein the medication sachets (201) are to be conveyed between the first endless belt (101) and the second endless belt (102) along the third path from the inlet (110) to the outlet (114), and means (119, 120, 121, 122, 124, 126) for rotating at least one of the first, second or third guide rollers (103, 104, 105). The invention also relates to a medication dispenser (300).

## Description

### TECHNICAL FIELD OF THE INVENTION

The present invention relates to a device for conveying medication sachets according to a preamble of the appended independent claim. The invention also relates to a medication dispenser.

### BACKGROUND OF THE INVENTION

Various medication dispensers are known for automated dispensing of medications to a patient. Many of these medication dispensers use strips of prepackaged medication sachets, where each medication sachet contains the medications that the patient should take at a prescribed date and time. The medication dispenser separates the medication sachets from the strip and dispenses them to the patient one medication sachet at a time.

Various devices are known for conveying a strip of medication sachets as well as separated medication sachets inside a medication dispenser. These devices typically comprise foam rollers and belt conveyors. The handling of the medication sachets is generally difficult because their shape is irregular and they tear easily, and because they have a habit of staying in the shape in which they have been stored in the medication dispenser.

In one known device, a strip of medication sachets is moved by pushing or pulling the strip forwards through pairs of foam rollers. The elasticity of the foam rollers provides a good grip and enables medication sachets having different thicknesses to travel between the foam rollers. A problem associated with the foam rollers is, however, that they provide only a small area of contact while leaving most of the medication sachets exposed to the surroundings, which causes a risk of the medication sachets being stuck or ripped due to contact with the non-moving mechanics inside the medication dispenser.

In another known device, a strip of medication sachets or separated medication sachets are moved between two belt conveyors. A problem associated with the opposed belt conveyors is that the construction is mechanically complicated and requires either the use of elastic pulleys or moving axles to provide clearance for the medication sachets. Another problem associated with the opposed belt conveyors is that the medication sachets can easily escape from the conveying path. Yet another problem associated with the opposed belt conveyors is that the medication sachets can only be conveyed along a straight line.

### OBJECTIVES OF THE INVENTION

It is the main objective of the present invention to reduce or even eliminate the prior art problems presented above.

It is an objective of the present invention to provide a device for conveying medication sachets. In more detail, it is an objective of the invention to provide a device enabling to convey medication sachets in a reliable and controlled manner inside a medication dispenser. It is a further objective of the invention to provide a device enabling to convey a plurality of medication sachets simultaneously.

It is also an objective of the invention to provide a medication dispenser for dispensing medications to a patient.

In order to realise the above-mentioned objectives, the device according to the invention is characterised by what is presented in the characterising portion of the appended independent claim. Advantageous embodiments of the invention are described in the dependent claims.

### DESCRIPTION OF THE INVENTION

A device for conveying medication sachets according to the invention comprises an inlet for receiving the medication sachets to be conveyed, an outlet for discharging the conveyed medication sachets, a first endless belt, a plurality of first guide elements arranged to guide the first endless belt along a first path, at least one of the first guide elements being a first guide roller, a second endless belt, a plurality of second guide elements arranged to guide the second endless belt along a second path, at least one of the second guide elements being a second guide roller, a plurality of third guide rollers arranged to guide the first endless belt and the second endless belt along a third path, wherein the medication sachets are to be conveyed between the first endless belt and the second endless belt along the third path from the inlet to the outlet, and means for rotating at least one of the first, second or third guide rollers.

The device according to the invention can be used in a medication dispenser for conveying medication sachets. The device can convey a strip of medication sachets as well as separated medication sachets. The strip of medication sachets comprises medication sachets attached one to another, where each medication sachet contains the medications that a patient should take at a prescribed date and time.

The device according to the invention has two endless belts between which the medication sachets are conveyed from the inlet to the outlet. The first guide elements are in contact with the first endless belt and arranged to guide the first endless belt along the first path. The first guide elements can be, for example, guide rollers, slide bars, or slide surfaces. In the device according to the invention, at least one of the first guide elements is a first guide roller. Preferably, all the first guide elements are first guide rollers. The second guide elements are in contact with the second endless belt and arranged to guide the second endless belt along the second path. The second guide elements can be, for example, guide rollers, slide bars, or slide surfaces. In the device according to the invention, at least one of the second guide elements is a second guide roller. Preferably, all the second guide elements are second guide rollers. The third guide rollers are arranged to guide the first endless belt and the second endless belt together along the third path. The first endless belt and the second endless belt are driven by rotating at least one of the first guide rollers, or at least one of the second guide rollers, or at least one of the third guide rollers with the rotating means. The other guide rollers are rotated by the moving first and second endless belts. Preferably, the rotating means is arranged to rotate at least one of the third guide rollers. This has the advantage of the first endless belt and the second endless belt automatically moving at the same speed without a need to synchronise their movements.

The medication sachets to be conveyed are received one after another into the device through the inlet. From the inlet the medication sachets are conveyed between the first endless belt and the second endless along the third path to the outlet. The medication sachets travel between the first endless belt and the second endless belt around the third guide rollers. Since the medication sachets are encapsulated between the first endless belt and the second endless belt, their contact with the surrounding mechanics is prevented. The medication sachets are discharged out of the device through the outlet.

The number of the guide elements and rollers, and the paths of the endless belts can be chosen according to the space constraints and the desired length of the third path. The number of the first and second guide elements can be, for example, 4-15, and the number of the third guide rollers can be, for example, 2-8. The diameter of the first and second guide rollers can be, for example, 0.5-5 cm, and the diameter of the third guide rollers can be, for example, 2-20 cm. The guide elements and rollers can be made of, for example, aluminium, steel, or polymer. The first endless belt and the second endless belt can be made of, for example, silicone, polyurethane or other elastic material with favourable friction properties.

The device may comprise a housing or a frame to which the guide elements and rollers, and the rotating means are supported. The housing or the frame can be made of, for example, aluminium, steel, or polymer.

An advantage of the device according to the invention is that it enables to convey medication sachets in a reliable and controlled manner. The device protects medication sachets from damage, tear or getting stuck on their way by clamping the medication sachets between two endless belts that are routed around guide elements and rollers. The medication sachets are fully protected from the surroundings when clamped between the endless belts that move at the same speed as the medication sachets.

Another advantage of the device according to the invention is that it enables to convey a plurality of medication sachets simultaneously, which means that it can store medication sachets while conveying them. The path of the endless belts can be convoluted or bent to enable storing a certain amount of medication sachets in the device or to take the medication sachets from a place to another via the desired path.

According to an embodiment of the invention the means for rotating at least one of the first, second or third guide rollers comprises an electric motor coupled to said at least one guide roller. The electric motor may be directly coupled to an end of a shaft of one of the guide rollers. Alternatively, or in addition, the electric motor may be coupled to an end of a shaft of one or more guide rollers with gear wheels, or with cogged belt pulleys and cogged belts. Preferably, the electric motor is coupled to at least one of the third guide rollers.

According to an embodiment of the invention the device comprises an inlet roller arranged at the inlet in connection with one of the plurality of first guide elements. Preferably, said first guide element is a first guide roller. The medication sachets are received into the device between the inlet roller and said first guide element. The inlet roller is preferably free-rotating. The inlet roller can be suspended to the device with one or more springs or rubber bands, which pull the inlet roller toward the first guide element. The spring or the rubber band can be attached between a shaft of the inlet roller and a housing or a frame of the device. The diameter of the inlet roller can be, for example, 2-10 cm. An advantage of the inlet roller is that together with the first guide element it facilitates the receiving of the medication sachets into the device.

According to an embodiment of the invention the inlet roller is arranged to be moveable with respect to the first guide element in connection with the inlet roller so that the distance between the inlet roller and said first guide element can vary. This enables medication sachets having different thicknesses to easily travel between the inlet roller and the first guide element.

According to an embodiment of the invention the device comprises means for applying a force pulling the inlet roller toward the first guide element in connection with the inlet roller. This enables keeping the medication sachets in tight and continuous contact when they travel between the inlet roller and the first guide element, irrespective of their thicknesses. The means for applying a force may comprise one or more springs or rubber bands attached to a shaft of the inlet roller.

According to an embodiment of the invention the device comprises an outlet roller arranged at the outlet in connection with one of the plurality of first guide elements. Preferably, said first guide element is a first guide roller. The medication sachets are discharged out of the device between the outlet roller and said first guide element. The outlet roller is preferably free-rotating. The outlet roller can be suspended to the device with one or more springs or rubber bands, which pull the outlet roller toward the first guide element. The spring or the rubber band can be attached between a shaft of the outlet roller and a housing or a frame of the device. The diameter of the outlet roller can be, for example, 2-10 cm. An advantage of the outlet roller is that together with the first guide element it facilitates the discharging of the medication sachets out of the device.

According to an embodiment of the invention the outlet roller is arranged to be moveable with respect to the first guide element in connection with the outlet roller so that the distance between the outlet roller and said first guide element can vary. This enables medication sachets having different thicknesses to easily travel between the outlet roller and the first guide element.

According to an embodiment of the invention the device comprises means for applying a force pulling the outlet roller toward the first guide element in connection with the outlet roller. This enables keeping the medication sachets in tight and continuous contact when they travel between the outlet roller and the first guide element, irrespective of their thicknesses. The means for applying a force may comprise one or more springs or rubber bands attached to a shaft of the outlet roller.

According to an embodiment of the invention the inlet roller and/or the outlet roller are foam rollers. The elasticity of the foam roller provides a good grip and enables medication sachets having different thicknesses to easily travel between the foam roller and the first guide element. When foam rollers are used, the inlet and outlet rollers do not have to be moveable with respect to the first guide element. The foam rollers can be made of, for example, rubber, polyurethane foam, EPDM, or EVA.

According to an embodiment of the invention the length of the third path is 10-100 cm. Preferably, the length of the third path is 15-70 cm. Considering the medication sachets, this means that the device can simultaneously convey 2-10 medication sachets between the first endless belt and the second endless belt.

According to an embodiment of the invention the number of the third guide rollers is 2-8. Preferably, the number of the third guide rollers is 2-5.

According to an embodiment of the invention the diameter of the third guide rollers is 2-20 cm. Preferably, the diameter of the third guide rollers is 5-15 cm.

According to an embodiment of the invention the third guide rollers have a larger diameter at the centre than at the ends. An advantage of this is that it reduces the stress on the strip of medication sachets because the shape enables the edges of the strip to take a slight shortcut around the third guide rollers. Without this shape there is a risk that the edges of the strip could be damaged. The diameter of the third guide roller can be, for example, 2-15 mm larger at the centre than at the ends. Preferably, the third guide rollers are barrel-shaped.

According to an embodiment of the invention the width of the first endless belt and the second endless belt is 4-15 cm. Preferably, the width of the first endless belt and the second endless belt is 6-10 cm. The width of the first endless belt and the second endless belt is preferably chosen in such a manner that it is at least 2 mm larger than the width of a medication sachet.

According to an embodiment of the invention the first endless belt and the second endless belt are made of an elastic material. The elastic material enables medication sachets having different thicknesses to be easily conveyed between the first endless belt and the second endless belt without requiring the use of soft guide rollers or moving shafts. The elastic material can be, for example, silicone, polyurethane or other elastic material with favourable friction properties.

The present invention also relates to a medication dispenser for dispensing medications to a patient. The medication dispenser comprises a device according to the invention for conveying medication sachets.

The medication dispenser may comprise a chamber into which a strip of medication sachets can be inserted. The strip of medication sachets can be conveyed from the chamber to an outlet of the medication dispenser by using the device according to the invention and optionally one or more other conveying devices, such as a pair of rollers which are rotated in opposite directions by an electric motor. The medication dispenser can separate the medication sachets from the strip and dispense them to the patient one medication sachet at a time. For this purpose, the medication dispenser may comprise a cutting device that is configured to cut the strip in a transverse direction at seam areas between the medication sachets. The separated medication sachets can be conveyed to the outlet from which the patient can take the medication sachets.

According to an embodiment of the invention the medication dispenser comprises a first imaging device arranged in connection with the inlet and configured to capture at least one image of the medication sachet before it is conveyed to the inlet. The medication dispenser may comprise means for processing the at least one image captured with the first imaging device to determine patient and medication related information of the medication sachet. The patient and medication related information may contain a patient identifier (e.g. name and/or identification number), and dispense date and time. This information can be printed using a specific layout on the medication sachet, either directly on a surface of the medication sachet or on a label that is attached on the medication sachet. The processing means may comprise a processor and a memory including computer program code, the memory and the computer program code being configured to, with the processor, determine the patient and medication related information of the medication sachet.

According to an embodiment of the invention the medication dispenser comprises a second imaging device arranged in connection with the outlet and configured to capture at least one image of the medication sachet after it has been conveyed out of the outlet. The medication dispenser may comprise means for processing the at least one image captured with the second imaging device to detect a seam area between medication sachets. The cutting device can cut the strip of medication sachets based on this information. The processing means may comprise a processor and a memory including computer program code, the memory and the computer program code being configured to, with the processor, detect the seam area between the medication sachets.

The exemplary embodiments of the invention presented in this text are not interpreted to pose limitations to the applicability of the appended claims. The verb "to comprise" is used in this text as an open limitation that does not exclude the existence of also unrecited features. The features recited in the dependent claims are mutually freely combinable unless otherwise explicitly stated.

### BRIEF DESCRIPTION OF THE DRAWINGS

- Fig. 1: illustrates a cutaway view of a device according to an embodiment of the invention,
- fig. 2: illustrates a perspective view of the device of fig. 1, and
- fig. 3: illustrates a medication dispenser according to an embodiment of the invention.

### DETAILED DESCRIPTION OF THE DRAWINGS

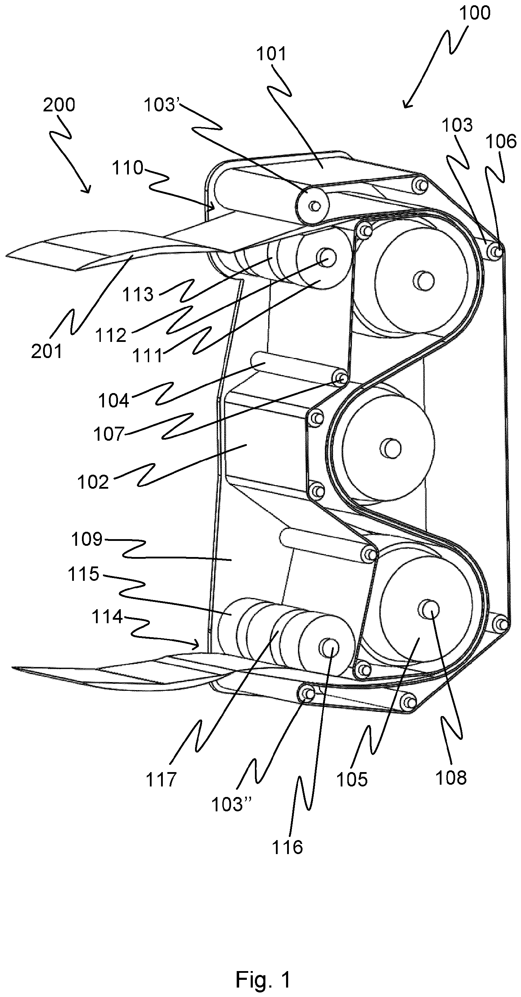

Fig. 1 illustrates a cutaway view of a device according to an embodiment of the invention. The device 100 is used for conveying a strip 200 of medication sachets 201. Each medication sachet 201 contains the medications that a patient should take at a prescribed date and time.

The device 100 comprises two endless belts 101 and 102, and three sets of guide rollers 103, 104 and 105. The guide rollers 103 are arranged to guide the endless belt 101 along a first path, and the guide rollers 104 are arranged to guide the endless belt 102 along a second path. The guide rollers 105 are arranged to guide the endless belts 101 and 102 together along a third path. The endless belts 101 and 102 are driven by rotating the top and bottom guide rollers 105 with a drive system (not shown in fig. 1). The middle guide roller 105 and the other guide rollers 103 and 104 are rotated by the moving endless belts 101 and 102. The guide rollers 103, 104 and 105 are supported at their shafts 106, 107 and 108 between two side plates 109 of which only one can be seen in fig. 1.

The medication sachets 201 to be conveyed are received one after another into the device 100 through an inlet 110. At the inlet 110, there is an inlet roller 111 that is arranged in connection with the guide roller 103'. The medication sachets 201 are received into the device 100 between the inlet roller 111 and the guide roller 103'. The inlet roller 111 is supported at its shaft 112 between the side plates 109. The inlet roller 111 has grooves 113. The inlet roller 111 is made of a foam material, which provides a good grip and enables medication sachets 201 having different thicknesses to easily travel between the inlet roller 111 and the guide roller 103'.

From the inlet 110 the medication sachets 201 are conveyed between the endless belts 101 and 102 around the guide rollers 105 to an outlet 114. Since the medication sachets 201 are encapsulated between the endless belts 101 and 102, their contact with the surrounding mechanics is prevented. The guide rollers 105 have a larger diameter at the centre than at the ends. This shape reduces the stress on the strip 200 because the edges of the strip 200 can take a slight shortcut around the guide rollers 105. The endless belts 101 and 102 are made of an elastic material, which enables medication sachets 201 having different thicknesses to be easily conveyed between the endless belts 101 and 102.

The medication sachets 201 are delivered out of the device 100 through the outlet 114. At the outlet 114, there is an outlet roller 115 that is arranged in connection with the guide roller 103". The medication sachets 201 are delivered out of the device 100 between the outlet roller 115 and the guide roller 103". The outlet roller 115 is supported at its shaft 116 between the side plates 109. The outlet roller 115 has grooves 117. The outlet roller 115 is made of a foam material, which provides a good grip and enables medication sachets 201 having different thicknesses to easily travel between the outlet roller 115 and the guide roller 103".

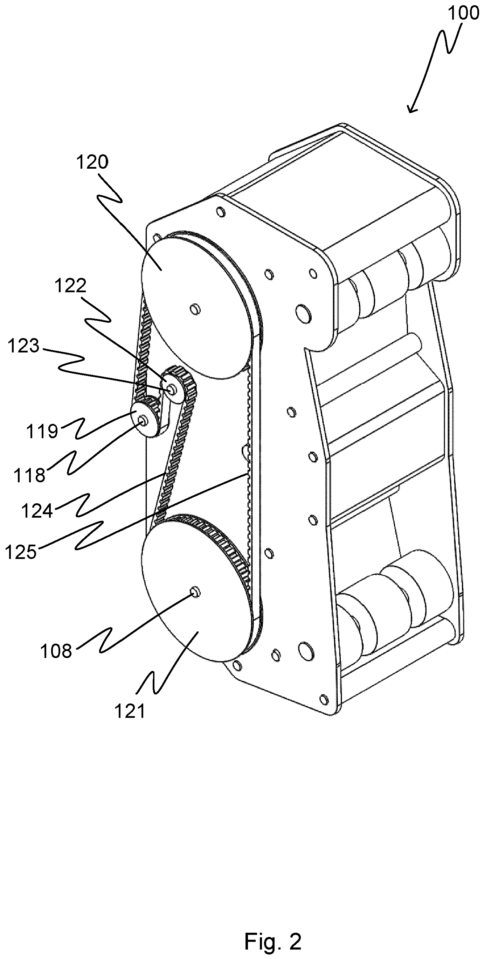

Fig. 2 illustrates a perspective view of the device of fig. 1. The device 100 is shown from a side to which the drive system that can rotate the top and bottom guide rollers 105 is arranged. The drive system comprises an electric motor (not shown in fig. 2) having an axle 118 to an end of which a cogged belt pulley 119 is attached. The drive system comprises two other cogged belt pulleys 120 and 121 which are attached to the ends of the shafts 108 of the top and bottom guide rollers 105, and a belt pulley 122 that is attached to an end of a shaft 123. A cogged belt 124 is arranged around the cogged belt pulleys 119, 120 and 121 and the belt pulley 122. The belt pulley 122 is used for tightening the cogged belt 124 to a desired tension and for guiding the cogged belt 124 in such a manner that enough teeth 125 of the cogged belt 124 are engaged with the cogged belt pulley 119. The cogged belt 124 is driven by rotating the cogged belt pulley 119 with the electric motor.

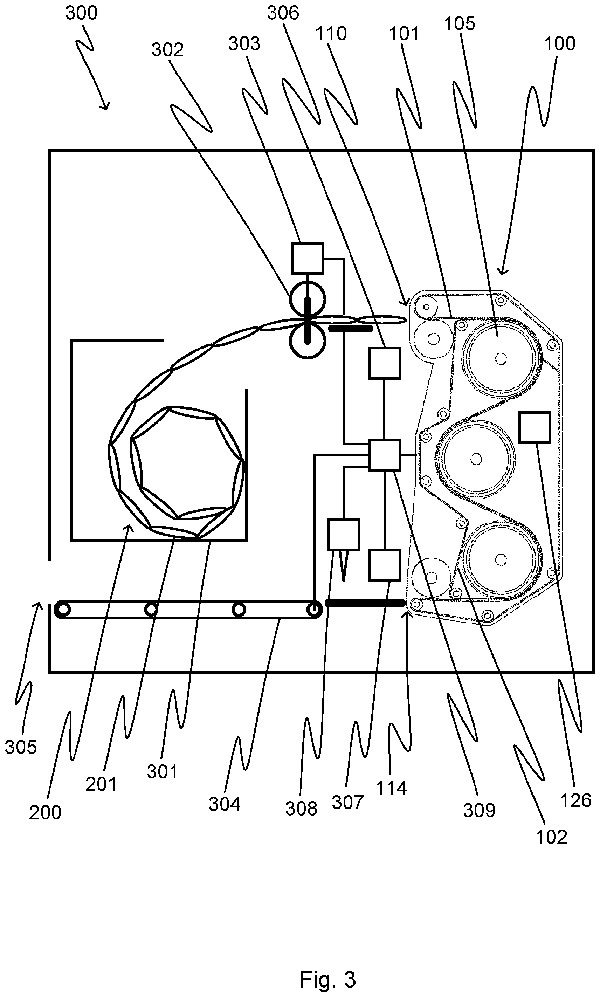

Fig. 3 illustrates a medication dispenser according to an embodiment of the invention for dispensing medications to a patient. The medication dispenser 300 comprises a chamber 301 into which a strip 200 of medication sachets 201 has been inserted. The medication dispenser 300 comprises a pair of rollers 302 driven by an electric motor 303 for conveying the medication sachets 201 from the chamber 301 to an inlet 110 of a conveying device 100. The conveying device 100 is similar to the one shown in fig. 1. The conveying device 100 conveys the medication sachets 201 between endless belts 101 and 102 from the inlet 110 to an outlet 114 of the conveying device 100. The medication dispenser 300 comprises a belt conveyor 304 for conveying the medication sachets 201 from the outlet 114 to an outlet 305 of the medication dispenser 300.

The medication dispenser 300 comprises an imaging device 306 that is arranged in connection with the inlet 110. The imaging device 306 is configured to capture at least one image of the medication sachet 201 before it is conveyed to the inlet 110 and to process the image(s) to determine patient and medication related information of the medication sachet 201. The medication dispenser comprises another imaging device 307 that is arranged in connection with the outlet 114. The imaging device 307 is configured to capture at least one image of the medication sachet 201 after it has been conveyed out of the outlet 114 and to process the image(s) to detect a seam area between medication sachets 201.

The medication dispenser 300 comprises a cutting device 308 that is arranged between the imaging device 307 and the belt conveyor 304. The cutting device 308 is configured to cut the strip 200 in a transverse direction at the seam areas that have been detected with the imaging device 307. The separated medication sachets 201 are conveyed with the belt conveyor 304 to the outlet 305 from which the patient can take the medication sachets 201.

The medication dispenser 300 comprises a control unit 309 that is connected to and configured to control the operation of the electric motor 303, the belt conveyor 304, the imaging devices 306 and 307, the cutting device 308, and an electric motor 126 of the conveying device 100. The electric motor 126 is used for driving the endless belts 101 and 102 by rotating the top and bottom guide rollers 105 of the conveying device 100.

Only advantageous exemplary embodiments of the invention are described in the figures. It is clear to a person skilled in the art that the invention is not restricted only to the examples presented above, but the invention may vary within the limits of the claims presented hereafter. Some possible embodiments of the invention are described in the dependent claims, and they are not to be considered to restrict the scope of protection of the invention as such.

## Claims

1. A device for conveying medication sachets, comprising:
- an inlet for receiving the medication sachets to be conveyed, and
- an outlet for discharging the conveyed medication sachets,
**characterised in that** the device comprises:
- a first endless belt,
- a plurality of first guide elements arranged to guide the first endless belt along a first path, at least one of the first guide elements being a first guide roller,
- a second endless belt,
- a plurality of second guide elements arranged to guide the second endless belt along a second path, at least one of the second guide elements being a second guide roller,
- a plurality of third guide rollers arranged to guide the first endless belt and the second endless belt along a third path, wherein the medication sachets are to be conveyed between the first endless belt and the second endless belt along the third path from the inlet to the outlet, and
- means for rotating at least one of the first, second or third guide rollers.

2. The device according to claim 1, **characterised in that** the means for rotating at least one of the first, second or third guide rollers comprises an electric motor coupled to said at least one guide roller.

3. The device according to claim 1 or 2, **characterised in that** the device comprises an inlet roller arranged at the inlet in connection with one of the plurality of first guide elements.

4. The device according to claim 3, **characterised in that** the inlet roller is arranged to be moveable with respect to the first guide element in connection with the inlet roller so that the distance between the inlet roller and said first guide element can vary.

5. The device according to claim 4, **characterised in that** the device comprises means for applying a force pulling the inlet roller toward the first guide element in connection with the inlet roller.

6. The device according to any of the preceding claims, **characterised in that** the device comprises an outlet roller arranged at the outlet in connection with one of the plurality of first guide elements.

7. The device according to claim 6, **characterised in that** the outlet roller is arranged to be moveable with respect to the first guide element in connection with the outlet roller so that the distance between the outlet roller and said first guide element can vary.

8. The device according to claim 7, **characterised in that** the device comprises means for applying a force pulling the outlet roller toward the first guide element in connection with the outlet roller.

9. The device according to any of claims 3 to 8, **characterised in that** the inlet roller and/or the outlet roller are foam rollers.

10. The device according to any of the preceding claims, **characterised in that** the length of the third path is 10-100 cm.

11. The device according to any of the preceding claims, **characterised in that** the number of the third guide rollers is 2-8.

12. The device according to any of the preceding claims, **characterised in that** the diameter of the third guide rollers is 2-20 cm.

13. The device according to any of the preceding claims, **characterised in that** the third guide rollers have a larger diameter at the centre than at the ends.

14. The device according to any of the preceding claims, **characterised in that** the first endless belt and the second endless belt are made of an elastic material.

15. A medication dispenser, **characterised in that** the medication dispenser comprises a device according to any of the preceding claims for conveying medication sachets.

## Amended claims

### Amended claims in accordance with Rule 137(2) EPC.

1. A device for conveying medication sachets, comprising:
- an inlet for receiving the medication sachets to be conveyed, and
- an outlet for discharging the conveyed medication sachets,
**characterised in that** the device comprises:
- a first endless belt,
- a plurality of first guide elements arranged to guide the first endless belt along a first path, at least one of the first guide elements being a first guide roller,
- a second endless belt,
- a plurality of second guide elements arranged to guide the second endless belt along a second path, at least one of the second guide elements being a second guide roller,
- a plurality of third guide rollers arranged to guide the first endless belt and the second endless belt along a third path, the third guide rollers having a larger diameter at the centre than at the ends, wherein the medication sachets are to be conveyed between the first endless belt and the second endless belt along the third path from the inlet to the outlet, and
- means for rotating at least one of the first, second or third guide rollers.

2. The device according to claim 1, **characterised in that** the means for rotating at least one of the first, second or third guide rollers comprises an electric motor coupled to said at least one guide roller.

3. The device according to claim 1 or 2, **characterised in that** the device comprises an inlet roller arranged at the inlet in connection with one of the plurality of first guide elements.

4. The device according to claim 3, **characterised in that** the inlet roller is arranged to be moveable with respect to the first guide element in connection with the inlet roller so that the distance between the inlet roller and said first guide element can vary.

5. The device according to claim 4, **characterised in that** the device comprises means for applying a force pulling the inlet roller toward the first guide element in connection with the inlet roller.

6. The device according to any of the preceding claims, **characterised in that** the device comprises an outlet roller arranged at the outlet in connection with one of the plurality of first guide elements.

7. The device according to claim 6, **characterised in that** the outlet roller is arranged to be moveable with respect to the first guide element in connection with the outlet roller so that the distance between the outlet roller and said first guide element can vary.

8. The device according to claim 7, **characterised in that** the device comprises means for applying a force pulling the outlet roller toward the first guide element in connection with the outlet roller.

9. The device according to any of claims 3 to 8, **characterised in that** the inlet roller and/or the outlet roller are foam rollers.

10. The device according to any of the preceding claims, **characterised in that** the length of the third path is 10-100 cm.

11. The device according to any of the preceding claims, **characterised in that** the number of the third guide rollers is 2-8.

12. The device according to any of the preceding claims, **characterised in that** the diameter of the third guide rollers is 2-20 cm.

13. The device according to any of the preceding claims, **characterised in that** the first endless belt and the second endless belt are made of an elastic material.

14. A medication dispenser, **characterised in that** the medication dispenser comprises a device according to any of the preceding claims for conveying medication sachets.
